# EUROPEAN PATENT SPECIFICATION

(11) **EP 4 633 476 B1**
(45) Date of publication and mention of the grant of the patent: **15.04.2026**
(21) Application number: 23821970.3
(22) Date of filing: 08.12.2023
(51) Int. Cl.: A61B 8/00

(54) **ULTRASOUND PROBE WITH SINGLE ENDED PROBE CONNECTOR**
ULTRASCHALLSONDE MIT EINSEITIGEM SONDENVERBINDER
SONDE À ULTRASONS À CONNECTEUR DE SONDE À EXTRÉMITÉ UNIQUE

(30) Priority: 15.12.2022 US 202263432901 P
(43) Date of publication of application: 22.10.2025
(73) Proprietor: Koninklijke Philips N.V., 5656 AG Eindhoven (NL)
(72) Inventor: SAVORD, Bernard Joseph, 5656 AG Eindhoven (NL); FAZIOLI, Theodore, 5656 AG Eindhoven (NL)
(74) Representative: Philips Intellectual Property & Standards
(86) International application number: PCT/EP2023/084888
(87) International publication number: WO 2024/126286

(56) References cited:
- US-A1- 2005 061 536
- US-A1- 2021 007 717
- ANONYMOUS: "AD9525 - Low Jitter Clock Generator with Eight LVPECL Outputs", 1 January 2013 (2013-01-01), pages 1 - 48, XP055509738, Retrieved from the Internet <URL:http://www.analog.com/media/en/technical-documentation/data-sheets/AD9525.pdf> [retrieved on 20180925]
- ANONYMOUS ED - ANONYMOUS: "IEEE guide for the installation of electrical equipment to minimize electrical noise inputs to controllers from external sources", 1 October 1982, IEEE STANDARD; [IEEE STANDARD], IEEE, PISCATAWAY, NJ, USA, XP017600773
- REFEREX, 1 January 2002 (2002-01-01), XP040425795

## Description

### BACKGROUND

Ultrasound imaging has become indispensable for many medical imaging applications. An ultrasound imaging system typically includes an ultrasound probe and a (host) processing system. The probe may include an array of ultrasound transducer elements configured to emit acoustic waves through a patient's body and to receive echo signals as the acoustic waves are reflected from the tissues, organs and other structures. The timing and strength of the echo signals generally correspond to the size, shape, and mass of the structures in the patient's body, images of which are displayed to a user of the ultrasound imaging system.

Modern ultrasound imaging systems may include digital probes that digitize the echo signals from the transducer elements within the probes themselves. The digital probes send high speed digital serial data to the host system, which requires very high data bandwidths. Since analog processing channels are not required in the base system connected to digital probes, image quality is not constrained by the ultrasound imaging system.

The high speed digital serial data are communicated between the digital probe and the host system typically using differential signals. Generally, when using differential signals, grounding does not matter, since ground noise couples equally into both lines and is subtracted out. Shielded twisted pairs (STPs) are used for sending the differential signals, in which a pair of separately shielded wires transmit each differential signal. Ultrasound systems include several channels and corresponding STPs for transmitting digital echo data streams from the ultrasound probe to the host system to adequately support large, overall digital data bandwidths. Therefore, the probe cable connecting the ultrasound probe to the host system necessarily has a large diameter and is inflexible due to the large number of STPs that it houses. Such a large, stiff cable causes the user (e.g., sonographer) to experience more difficulty when attempting to manipulate the ultrasound probe attached to the probe cable. Alternatively, pairs of coaxial cables may be include in the cable assembly to transmit the differential signals. However, tight tolerances on the skew are required between the individual wires in each pair of coaxial cables, making this solution impractical.

US 2021/007717 A1 describes an ultrasound image system including a communication link including at least one data lane in communication with a host system.

US 2005/061536 A1 describes methods and systems for reducing crosstalk during continuous wave ultrasound data acquisition.

Anonymous, *"IEEE guide for the installation of electrical equipment to minimize electrical noise inputs to controllers from external sources ",* describes a guide for the installation and operation of industrial controllers to minimize the disturbing effects of electrical noise on these controllers.

### SUMMARY OF THE DISCLOSURE

The invention is set out in the appended set of claims.

According to a representative embodiment, there is provided an ultrasound imaging system according to claim 1.

### BRIEF DESCRIPTION OF THE DRAWINGS

The representative embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1 is a simplified schematic diagram of an ultrasound imaging system including a single ended data link between a digital ultrasound probe and a host, according to a representative embodiment.
FIG. 2 is a simplified block diagram of an ultrasound imaging system including a digital ultrasound probe, a host system, and a single ended data link connecting the digital ultrasound probe and the host, according to a representative embodiment.
FIG. 3 is a simplified block diagram of the digital ultrasound probe in the ultrasound imaging system of FIG. 3, according to a representative embodiment.
FIG. 4 is a simplified block diagram of the host system in the ultrasound imaging system of FIG. 3, according to a representative embodiment.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

As used in the specification and appended claims, and in addition to their ordinary meanings, the term "approximately" mean to with acceptable limits or degree. For example, "approximately 20 GHz" means one of ordinary skill in the art would consider the signal to be 20 GHz within reasonable measure.

As used in the specification and appended claims, in addition to their ordinary meanings, the term "substantially" means within acceptable limits or degree. For example, the "plurality of transducer ports are substantially the same" means one of ordinary skill in the art would consider the plurality of transducer ports to be the same.

According to various embodiments, a probe cable connecting the digital ultrasound probe and the host system in an ultrasound imaging system incorporates single ended data links for transmitting digital echo data. This enables use of coaxial cables for the data links instead of shielded twisted pairs (STPs), resulting in half as many wires in the probe cable. That is, transmission of differential signals requires use of STPs, as discussed above. Single ended communications, however, are able use simple coaxial cables with a single shield. Since there is only one cable instead of two within the shield, the overall diameter of a coaxial cable is smaller than that of an STP. Therefore, when multiple coaxial cables are bundled together in the probe cable connecting the ultrasound probe and the host system, the overall diameter of the probe cable is significantly reduced and flexibility is increased.

Generally, a single wire without a return path does not work well for transmitting digital signals. So, in order to incorporate the single ended data links, terminations on unused halves of the differential signals provide balance to the driver in the ultrasound probe and receiver in the host system, as well as minimize coupling from reflections in the unused halves. Also, the unused halves are terminated in a quiet environment away from the receiving host system to eliminate common mode noise from the noisy digital environment.

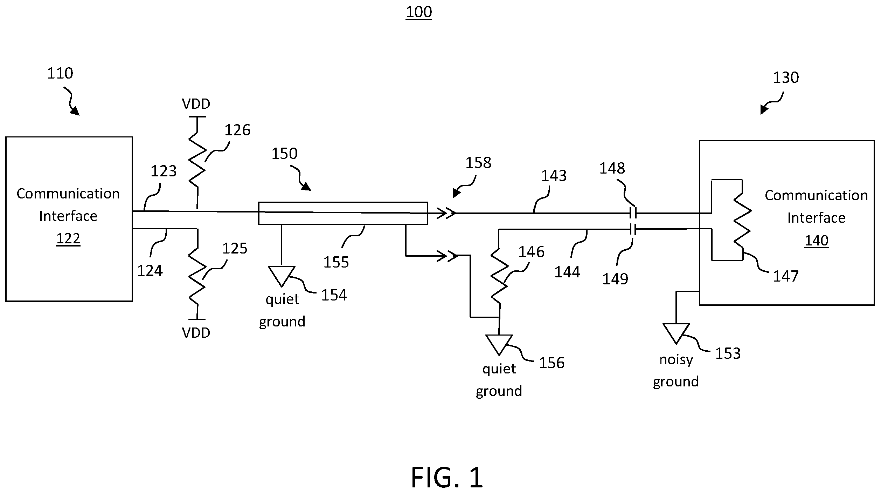

FIG. 1 is a simplified schematic diagram of an ultrasound imaging system including a single ended data link between a digital ultrasound probe and a host, according to a representative embodiment.

Referring to FIG. 1, ultrasound imaging system 100 is used for scanning an area or volume of a patient's body using ultrasound imaging signals. The ultrasound imaging system 100 includes a digital ultrasound probe 110 and a host system 130 connected by cable assembly 150. The cable assembly 150 includes a probe cable that contains multiple single ended data links 155 for transmitting digital echo data streams from the ultrasound probe 110 to the host system 130, as discussed below. For ease of illustration, one single ended data link 155 is shown in the probe assembly 150, although it is understood that, in practice, multiple single ended data links 155 would be included in the cable assembly 150 for transmitting the digital echo data streams from multiple channels of the ultrasound probe 110, respectively, to the host system 130. The total number (L) of single ended data links 155 in the probe cable assembly 150 is sufficient to support high data bandwidths of the digital echo data streams. The probe assembly 150 may also include conductors for transmission of power and control signals from the host system 130 to the ultrasound probe 110, as discussed below. These conductors may include wires, STPs, or any other suitable means of transferring data, signals or power, and may be differential links or single ended links. For example, power may be sent over hook-up wire and control data maybe sent as single ended low voltage complementary metal oxide semiconductor (LVCMOS) or differential low voltage differential signaling (LVDS).

The single ended data link 155 may be a coaxial cable, such as a 50 ohm coaxial cable, for example. Use of the single ended data links 155, as compared to conventional differential data links (e.g., STPs), results in fewer physical interconnects between the ultrasound probe 110 and the host system 130, thereby reducing diameter and stiffness of the probe assembly 150. The single ended data link 155 is connected to quiet ground 154 of the probe assembly 150, and is connected to quiet ground 156 of the host system 130 via system connector 158.

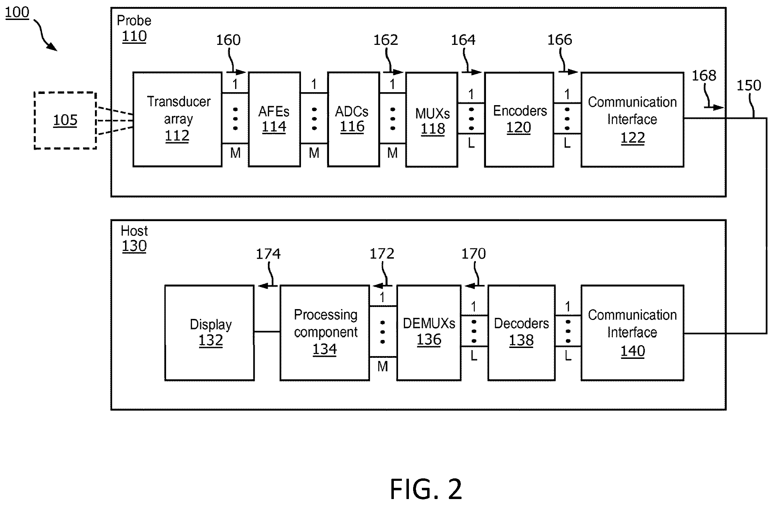

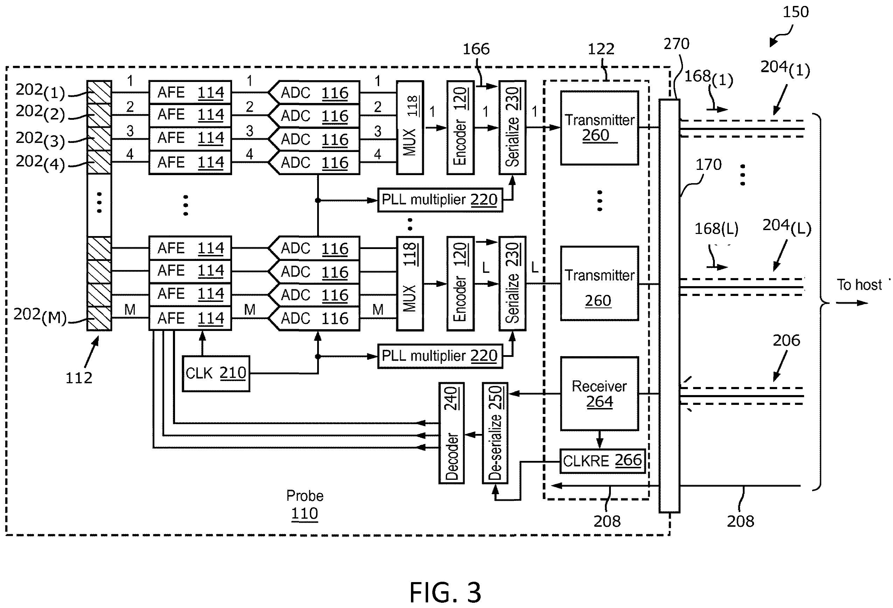

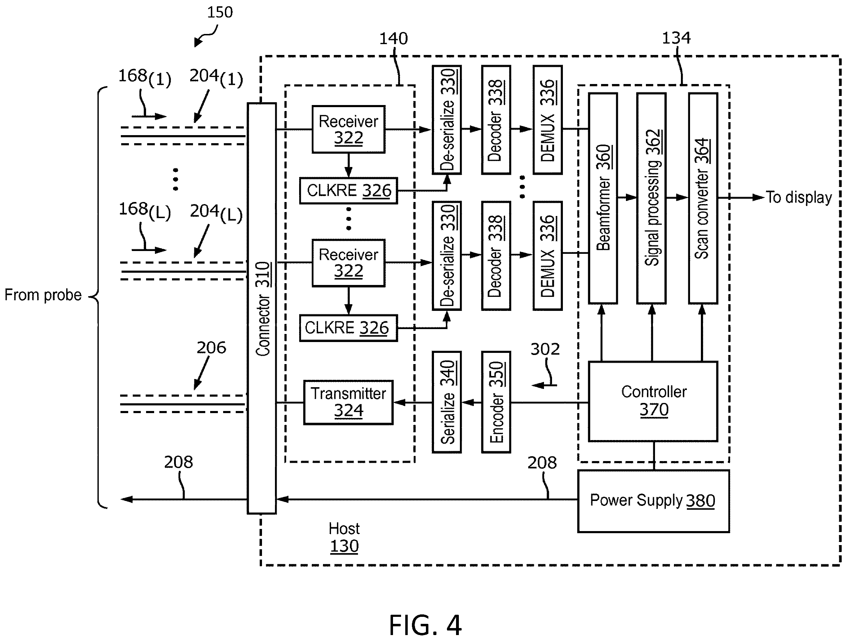

The ultrasound probe 110 includes a communication interface 122, discussed below in detail with reference to FIGs. 2 and 3. The communication interface 122 may include an application specific integrated circuit (ASIC), to which the single ended data link 155 connects on the probe side. The ASIC may include a current mode logic (CML) cable driver, for example, for outputting the digital echo data streams at high data bandwidths. The host system 130 similarly includes a communication interface 140, also discussed below in detail with reference to FIGs. 2 and 4. The communication interface 140 may include a field programmable gate array (FPGA), to which the single ended data link 155 connects on the system side, for receiving the digital echo data streams.

In the depicted embodiment, the host system 130 includes a pair of host conductors 143 and 144 for transmitting the digital echo data streams within the host system 130. The host conductors 143 and 144 may be 100 ohm PCB traces, micro-strip traces, or strip lines, for example. The host conductors 143 and 144 are paired because they are generally designed to accommodate differential signals. However, according to the depicted embodiment, the host conductors 143 and 144 are configured for single ended data transmission by connecting the host conductor 143 to the single ended data link 155 to receive the digital echo data streams and connecting the host conductor 144 to the quiet ground 156 through first termination resistance 146 (e.g., a 50 ohm resistor). The used host conductor 143 is terminated to the unused host conductor 144 through resistance 147 (e.g., a 100 ohm resistor) in the communication interface 140. The host conductor 143 is configured to pass the digital echo data streams from the single ended data link 155 to the host system 130, while the host conductor 144 is grounded. Therefore, the host conductor 143 may be referred to as the "used host conductor," while the host conductor 144 may be referred to the "unused host conductor."

Likewise, in the depicted embodiment, the ultrasound probe 110 includes a pair of probe conductors 123 and 124 for transmitting the digital echo data streams within the ultrasound probe 110. The probe conductors 123 and 124 may comprise 50 ohm printed circuit board (PCB) traces, micro-strip traces, or strip lines, for example. The probe conductors 123 and 124 are paired because they are generally designed to accommodate differential signals. However, according to the depicted embodiment, the probe conductors 123 and 124 are configured for single ended data transmission by connecting the probe conductor 124 to a voltage source (e.g., VDD) through second termination resistance 125 (e.g., a 25 ohm resistor) and connecting the probe conductor 123 to the voltage source through third termination resistance 126 (e.g., a 50 ohm resistor). The probe conductor 123 is configured to transmit the digital echo data streams to the single ended data link 155. Therefore, the probe conductor 123 may be referred to as the "used probe conductor," while the probe conductor 124 may be referred to the "unused probe conductor."

The first, second and third termination resistances 146, 125 and 126 minimize intersymbol interference from transmission line reflections in the single ended data link. The respective values of the first, second and third termination resistances 146, 125 and 126 are selected to preserve source and load impedances as they would have been seen by the communication interfaces 122 and 140 in a conventional differential arrangement.

The communication interface 140 is also connected to noisy ground 153, which represents common mode digital noise. However, as mentioned above, the unused host conductor 144 is connected to the quiet ground 156 at a location outside the noisy digital environment. This enables rejection of the common mode digital noise in the single ended configuration, which would not be necessary in a differential configuration.

The ultrasound imaging system 100 may also include AC couplers 148 and 149 in the host conductors 143 and 144, respectively, to provide AC coupling. The AC coupling provides a known signal threshold. Therefore, the AC coupling preserves DC biasing within the communication interface 140 (e.g., FPGA) to obtain a threshold centered with respect to the digital echo data streams.

FIG. 2 is a simplified block diagram of an ultrasound imaging system including a digital ultrasound probe, a host system, and a single ended data link connecting the digital ultrasound probe and the host, according to a representative embodiment. The ultrasound imaging system is used for scanning a region, area, or volume of a patient's body. Portions of the ultrasound imaging system are described, for example, in U.S. Patent App. Pub. No. 2021/0007717, titled "Digital Ultrasound Cable and Associated Devices, Systems, and Methods," filed August 13, 2020.

Referring to FIG. 2, the ultrasound imaging system 100 includes the ultrasound probe 110 in communication with the host system 130 over the cable assembly 150, including includes single ended data links. At a high level, the ultrasound probe 110 emits ultrasound waves towards a medium 105 (e.g., an anatomical object in a patient's body) and receives echoes that are reflected in the medium 105. The ultrasound probe 110 transmits per-channel echo signals digitally over the cable assembly 150 to the host system 130 for processing and image display. The ultrasound probe 110 may be in any suitable form for imaging various body parts of a patient while positioned inside or outside of the patient's body. For example, the ultrasound probe 110 may be in the form of a catheter, a transesophageal echocardiography (TEE) probe, a transthoracic echocardiography (TTE) probe, an endo-cavity probe, an intraluminal probe, a handheld ultrasound scanner, or a patch-based ultrasound device.

In the depicted embodiment, the ultrasound probe 110 includes a transducer array 112, analog front ends (AFEs) 114, analog-to-digital converters (ADCs) 116, multiplexers (MUXs) 118, encoders 120, and a communication interface 122. The AFEs 114, the ADCs 116, the MUXs 118, the encoders 120, and/or the communication interface 122 may be incorporated in one or more ASICs, discussed above. The host system 130 includes a display 132, a processing component 134, de-multiplexers (DEMUXs) 136, decoders 138, and a communication interface 140. The DEMUXs 136, the decoders 138 and/or the communication interface 140 may be incorporated in one or more FPGAs, discussed above.

The transducer array 112 emits ultrasound signals towards the medium 105 and receives ultrasound echo signals reflected from the medium 105 back to the transducer array 112, and converts the ultrasound echo signals to electrical analog echo signals 160. The transducer array 112 may include acoustic elements arranged in a one-dimensional (1D) array or in a two-dimensional (2D) array. The acoustic elements may be referred to as transducer elements, and may be capacitive micromachined ultrasonic transducers (CMUTs) or piezoelectric transducers formed of materials such as PZT or PVDF, for example. Each transducer element may emit ultrasound waves towards the medium 105 and may receive the ultrasound echo signals as the ultrasound waves are reflected back from the medium 105. For example, the transducer array 112 may include M transducer elements for producing M analog echo signals 160 in corresponding M channels. In some embodiments, M may be 2, 16, 64, 128, 192, or greater than 192, for example.

The AFEs 114 are coupled to the transducer array 112 via the M channels. Each AFE 114 may be coupled to one transducer element in the transducer array 112. The AFEs 114 may include circuitry configured to provide high voltage excitations and gain controls. The high voltage excitations can trigger ultrasound wave emissions at the transducer elements. The gain controls can provide low-noise pre-amplification to the received echoes.

The ADCs 116 are coupled to the AFEs 114 via the M channels. Each ADC 116 may be coupled to one AFE 114 and configured to convert a corresponding analog echo signal 160 into a digital echo data signal 162. In some embodiments, the ADCs 116 are successive approximation type ADCs. The successive approximation ADC architecture may provide high-performance and lower-power consumption, and thus may keep total power dissipation of the ultrasound probe 110 to be within a thermal budget of the ultrasound probe 110. Each digital echo data signal 162 includes digital samples representing the waveforms of a corresponding analog echo signal. The M digital echo data signals 162 may be referred to as per-channel ultrasound digital echo data streams or channelized ultrasound digital echo data streams.

The MUXs 118 are coupled to the ADCs 116 via the M channels. Each MUX 118 may be coupled to a subset of the ADCs 116 via a corresponding subset of signal lines and configured to multiplex a corresponding subset of the channelized digital echo data signals 162. As an example, the ADCs 116 may be grouped into L subsets, in which case the ultrasound probe 110 may include L MUXs 118 producing L multiplexed digital echo data streams 164, where L is less than or equal to M. However, in an embodiment, MUXs 118 are not included, in which case the number of signal lines is M. The MUXs 118 are digital MUXs and may be implemented using a combination of hardware components and software components.

The encoders 120 are coupled to the MUXs 118 via the L signal lines. Each encoder 120 may be coupled to one MUX 118 and configured to encode a corresponding multiplexed digital echo data stream 164 into an encoded digital echo data stream 166. The encoders 120 can be implemented using a combination of hardware components and software components. In some embodiments, the encoders 120 may implement an 8b/l0b encoding algorithm, for example, as described in U.S. Patent No. 4,486,739, filed June 30, 1982, which is hereby incorporated by reference in its entirety. The 8b/l0b encoding maps an 8-bit input data unit into 10-bit output symbols. The 8b/l0b encoding maximizes the number of bit-transitions in the encoded digital echo data stream 164 and can provide a minimal direct current (DC) component. The encoders 120 can produce L encoded digital echo data streams 166.

The communication interface 122 is coupled to the encoders 120 via L signal lines. The communication interface 122 is configured to transmit the L encoded digital echo data streams 166 to the host system 130 via the cable assembly 150. The communication interface 122 may include a combination of hardware components and software components configured to generate digital data signals 168 carrying the encoded digital echo data streams 166 for transmission over the cable assembly 150. The cable assembly 150 includes a probe cable, which contains L single ended data links, as discussed above, for transferring the digital data signals 168 to the host system 130.

The host system 130 may be any suitable computing and display device, such as a workstation, a personal computer (PC), a laptop, a tablet, a mobile phone, or a patient monitor, for example. In some embodiments, the host system 130 may be located on a moveable cart. At the host system 130, communication interface 140 may receive the digital data signals 168 from the ultrasound probe 110 via the cable assembly 150. The communication interface 140 may include a combination of hardware components and software components. The communication interface 140 may be substantially similar to the communication interface 122 in the ultrasound probe 110.

The decoders 138 are coupled to the communication interface 140 via L signal lines. Each decoder 138 is configured to receive a digital data signal 168 from one of the L signal lines and perform decoding on the digital data signal 168 to recover a decoded data stream 170. The decoding may be performed according to the encoding algorithm (e.g., according to the 8b/10b encoding) used by the encoders 120 at the ultrasound probe 110. The decoders 138 may be implemented using a combination of hardware components and software components. The decoders 138 may produce L decoded data streams 170.

The DEMUXs 136 are coupled to the decoders 138 via L signal lines. Each DEMUX 136 may be coupled to one decoder 138 and configured to de-multiplex a corresponding decoded data stream 170 into data streams 172 corresponding to the per-channel ultrasound echo data streams at the output of the ADCs 116. The DEMUXs 136 may produce M de-multiplexed ultrasound digital echo data streams 172 corresponding to the ultrasound digital echo data signals 162.

The processing component 134 is coupled to the DEMUXs 136 via M signal lines. The processing component 134 may be configured to generate image signals 174 for display to a user and/or perform image processing and image analysis for various diagnostic modalities or ultrasound types (B mode, CW Doppler, etc.). The processing component 134 may include a general purpose computer, a computer processor, a microprocessor, a graphics processing unit (GPU), a central processing unit (CPU), a digital signal processor (DSP), a microcontroller, a state machine, programmable logic device, field programmable gate arrays (FPGAs), application specific integrated circuits (ASICs), systems on a chip (SOC), or combinations thereof. The processing component 134 may also be implemented as a combination of computing devices, e.g., a combination of a DSP and a microprocessor, a GPU and a microprocessor, multiple microprocessors, one or more microprocessors in conjunction with a DSP core, or any other such configuration. The processing component 134 may be configured to generate image signals 174 from the de-multiplexed ultrasound digital echo data streams 172 and/or perform image processing and image analysis for various diagnostic modalities.

The display 132 is coupled to the processing component 134. The display 132 may include a monitor, a touch-screen, a television, a liquid crystal display (LCD), a light emitting diode (LED) display, a flat panel display, a solid-state display, a cathode ray tube (CRT) display, or any suitable display, for example. The display 132 is configured to display images and/or diagnostic results processed by the processing component 134. The host system 130 may further include a user interface (not shown) configured to receive user inputs for controlling the ultrasound imaging system 100. The user interface may include one or more of a mouse, a keyboard, a mouse, a trackball, a joystick, a microphone, a video camera, a touchpad, a touchscreen, voice or gesture recognition captured by a microphone and a video camera, for example. All or part of the user interface may be incorporated with the display 132 as a graphical user interface (GUI) for displaying and receiving information to and from the user.

While FIG. 2 is described in the context of transferring detected ultrasound digital echo data from the ultrasound probe 110 to the host system 130 for display, the host system 130 may generate controls for configuring the ultrasound probe 110, for example, the excitations of the transducer elements at the transducer array 112, as described below in greater detail.

FIGs. 3 and 4 collectively provide a more detailed view of the ultrasound imaging system 100, including transmission paths from the ultrasound probe 110 to the host system 130, and from the host system 130 to the ultrasound probe 110. FIG. 3 is a simplified block diagram of the ultrasound probe 110 in the ultrasound imaging system 100, according to a representative embodiment, providing a more detailed view of the internal components in the ultrasound probe 110. FIG. 4 is a simplified block diagram of the host system 130 in the ultrasound imaging system 100, according to a representative embodiment, providing a more detailed view of the internal components in the host system 130.

Referring to FIG. 3, the ultrasound probe 110 further includes a clock (CLK) 210, L phase-locked loop (PLL) multipliers 220, L serializing components 230, a decoder 240, a de-serializing component 250, and a connector 270. The communication interface 122 includes L transmitters 260, a receiver 264, and a clock recovery (CLKRE) component 266. The connector 270 couples the communication interface 122 to the cable assembly 150.

As shown in FIG. 4, the host system 130 further includes a connector 310, L de-serializing components 330, a serializing component 340, an encoder 350 and a power supply 380. The communication interface 140 may include L receivers 322, a transmitter 324, and L CLKRE components 326. The processing component 134 may include a beamformer 360, a signal processing component 362, a scan converter 364, and a controller 370. The power supply 380 may provide power to the host system 130 and to the ultrasound probe 110.

As shown in FIGs. 3 and 4, the cable assembly 150 includes L single ended data links 204 (e.g., shown as 204(1) to 204( L) for transmitting signals to the host system 130. The cable assembly 150 may also include control and power conductors in data link 206 and power line 208. The data link 206 and the power line 208 may include one or multiple signal and/or power lines including conductors, STPs, or any other suitable means of transferring data, signals or power, and may be differential links or single ended links (as discussed above with regard to the single ended links 204), without departing from the scope of the present teaches. Generally, the data link 206 is for receiving control and other signals from the host system 130, and the power line 208 for receiving power from the power supply 380 in the host system 130.

The transmission path from the ultrasound probe 110 to the host system 130 may begin at the transducer array 112 shown in FIG. 3. As shown, the transducer array 112 includes M transducer elements 202, each connecting to an AFE 114. The transducer elements 202 are shown as 202(1) to 202(M).

The CLK 210 may function as a master clock in the ultrasound probe 110. The CLK 210 may provide a clock signal to the AFEs 114 and the ADCs 116. The ADCs 116 may be grouped into groups of four ADCs 116, and thus L may be M/4, for example. In other embodiments, the ADCs 116 may be grouped into groups of 2, 8, or more than 8. Each MUX 118 may be coupled to one group of ADCs 116.

Each serializing component 230 is coupled to the output of an encoder 120. As described above, the encoders 120 may encode an 8-bit input data unit into 10-bit output symbols, for example. The serializing component 230 may convert the output symbols (e.g., the encoded digital echo data streams 166) into a bit stream for transmission.

Each PLL multiplier 220 is coupled to the CLK 210 and a serializing component 230. The PLL multipliers 220 are configured to convert the frequency of the clock signal into a suitable frequency for operating the serializing components 230. As an example, the CLK 210 may provide a clock frequency of about 40 MHz and the ADCs 116 may be 12-bit ADCs. When the encoders 120 produce 10 bits of output for every 8 bits of input, the serializing component 230 is required to operate at a data rate of about 2.4 gigabits per second (Gbps). Thus, each PLL multiplier 220 may convert the 40 MHz clock signal into a 2.4 GHz clock signal for operating a corresponding serializing component 230.

Each transmitter 260 is coupled to one serializing component 230. The transmitter 260 may include circuitry for driving the cable assembly 150. The transmitters 260 may receive the encoded digital echo data streams 166 and generate digital data signals 168 carrying the encoded digital echo data streams 166 for transmission over the single ended data links 204. The digital data signals 168 are shown as 168(1) to l68(L), each corresponding to one of the encoders 120, respectively. The transmissions of the digital data signals 168(1) to l68(L) may occur simultaneously over corresponding single ended data links 204(1) to 204(L). In some embodiments, the transmitters 260 may implement a current mode logic (CML) physical layer for the transmissions, as described in greater detail herein.

Referring to the example described above, where the CLK 210 runs at 40 MHz, the ADCs 116 provides 12-bit samples, and the ADCs 116 are grouped into groups of 4. When the transducer array 112 includes 128 (e.g., M = 128) transducer elements 202, the cable assembly 150 may include 32 single ended data lanes, each with a data transfer rate of about 2.4 Gbps. Thus, the cable assembly 150 may provide a data transfer rate of about 76.8 Gbps, which is substantially the same as the data transfer rate enabled by conventional differential data links, e.g., comprising STPs.

As shown in FIG. 4, at the host system 130, the receivers 322 may receive the digital data signals 168 carrying the encoded, multiplexed channelized digital echo data streams via the single ended data links 204 of the cable assembly 150. Each CLKRE component 326 is coupled to a receiver 322 and a de-serializing component 330. The CLKRE component 326 is configured to recover a clock signal from the received digital data signal 168 and provide the clock signal to a corresponding receiver 322 and de-serializing component 330. The receivers 322 may recover the bit stream transmitted by the ultrasound probe 110 over corresponding single ended data links 204. The de-serializing components 330 may convert the recovered bit streams into symbols based on the bit-size of the output symbols produced by the encoder 120 at the ultrasound probe 110. For example, when the encoder 120 implements the 8b/l0b encoding, the de-serializing components 330 may form data in units of 10 bits. Thus, each de-serializing component 330 may produce a stream of 10-bits data and provide the data stream to a corresponding decoder 338 for decoding.

The decoders 338 and the DEMUXs 336 may operate as described above to recover the channelized digital echo data streams generated at the ultrasound probe 110. The beamformer 360 is configured to apply timing delays to the ultrasound echo channel data streams 172 to align the timings of the different channels and may sum the time-aligned ultrasound echo channel data streams to produce beamformed signals. The signal processing component 362 is configured to perform filtering and/or quadrature demodulation to condition the beamformed signals. The signal processing component 362 may perform analytic detection and/or any image processing techniques on the conditioned signals to produce image signals 174. The scan converter 364 is configured to convert the image signals 174 into images for display, for example, on the display 132. The controller 370 may control the operations of the beamformer 360, the signal processing component 362, and/or the scan converter 364.

The transmission path from the host system 130 to the ultrasound probe 110 may begin at the controller 370 of the host system 130 shown in FIG. 4. The controller 370 may further generate control data 302 for operating the transducer elements 202 at the transducer array 112, for example, for ultrasound wave emissions. At the host system 130, the encoder 350 is coupled to the controller 370. The encoder 350 may be substantially similar to the encoder 120. For example, the encoder 350 may encode the control data using the same encoding algorithm (e.g., the 8b 10b encoding algorithm) as the encoder 120. The serializing component 340 is coupled to the transmitter 324. The serializing component 340 may be substantially similar to the serializing component 230. For example, the serializing component 340 may convert the encoded control data stream into a bit stream. The transmitter 324 may be substantially similar to the transmitters 260. For example, the transmitter 324 may generate a digital signal carrying the encoded control data bit stream for transmission over the single ended data link 206.

At the ultrasound probe 110, the receiver 264 may receive the digital signal carrying the encoded control data bit stream from the host system 130. The CLKRE component 266 is coupled to the receiver 264. The receiver 264 may be substantially similar to the receiver 322. The CLKRE component 266 may be substantially similar to the CLKRE components 326. For example, the CLKRE component 266 may recover a clock signal from the received digital signal and the receiver 264 may recover the bit streams transmitted by the host system 130 from the receive signals. The de-serializing component 250 is coupled to the receiver 264 and the decoder 240. The de-serializing component 250 may be substantially similar to the de-serializing components 330. The decoder 240 may be substantially similar to the decoders 138. For example, the de-serializing component 250 may convert the bit stream into a data stream and the decoder 240 may perform decoding to recover the control data transmitted by the host system 130. The decoder 240 is coupled to the AFEs 114. For example, the control data may include excitation information for trigger ultrasound wave emissions at the transducer elements 202.

As can be seen, the inclusions of the ADCs 116 at the ultrasound probe 110 allows the transfer of per-channel digital ultrasound echo data channels from the ultrasound probe 110 to the host system 130 for maximum processing flexibility. The use of the MUXs 118 and the parallel multi-lane cable assembly 150 provides a high-speed data transfer rate that is at an order of magnitude higher than currently available standard digital communication protocols (e.g., USB 3.0).

In some embodiments, the required bandwidth over the cable assembly 150 may be reduced by including an analog sub-array processor at the ultrasound probe 110 between the AFEs 114 and the ADCs 116. The sub-array processor may perform partial beamforming to combine a subset of the analog echo signals 160. The partial beamforming can further reduce the number of signal lines (e.g., the data links 204) required in the transmission path between the ultrasound probe 110 and the host system 130 or reduce the required data transfer data for each single ended data link 204. The full beamforming can be performed at the host system 130.

In some embodiments, the required bandwidth over the cable assembly 150 may be reduced by including a digital partial beamformer at the ultrasound probe 110 between the ADCs 116 and the MUXs 118. The digital partial beamformer may perform partial beamforming to combine a subset of the ultrasound digital echo data signals 162. In such embodiments, the MUXs 118 can multiplex the partial beamformed ultrasound echo data streams for encoding by the encoders 120. Similar to the analog partial beamforming, the digital partial beamforming can further reduce the number of signal lines (e.g., the single ended data links 204) required in the transmission path between the ultrasound probe 110 and the host system 130 or reduce the required data transfer data for each single ended data link 204. The full beamforming may be performed at the host system 130.

In some embodiments, the required bandwidth over the cable assembly 150 may be reduced by including both the analog partial beamformer and the digital partial beamformer at the ultrasound probe 110 as described above.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems, such as in a cloud-based or other multi-site application. The term computing device should also be interpreted to include a collection or network of computing devices each including a processor or processors. Programs have software instructions performed by one or multiple processors that may be within the same computing device or which may be distributed across multiple computing devices.

The term "memory" as used herein encompasses a non-transitory computer-readable medium that stores instructions, which may communicate with each other and processor(s) via one or more buses. Memory stores instructions used to implement some or all aspects of methods and processes described herein. A memory may include a cache memory (e.g., a cache memory of the processor), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. The term "non-transitory" is to be interpreted not as an eternal characteristic of a state, but as a characteristic of a state that will last for a period, and specifically disavows fleeting characteristics such as characteristics of a carrier wave or signal or other forms that exist only transitorily in any place at any time.

Although methods, systems and components for implementing imaging protocols have been described with reference to several exemplary embodiments, it is understood that the words that have been used are words of description and illustration, rather than words of limitation. The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the scope of the appended claims. Thus, to the maximum extent allowed by law, the scope of the present disclosure is to be determined by the broadest permissible interpretation of the following claims and their equivalents and shall not be restricted or limited by the foregoing detailed description.

The Abstract of the Disclosure is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

## Claims

1. A ultrasound imaging system (100), comprising:
an ultrasound probe (110) configured to generate a digital echo data stream responsive to ultrasound signals reflected in a medium; **characterized in that** the system comprises:
a cable assembly (150) comprising a plurality of single ended data links (155), wherein the cable assembly is configured to communicate the ultrasound echo data stream to a host system (130) via the plurality of single ended data links;
and **in that** the host system comprises:
a plurality of host conductors, wherein used host conductors of the plurality of host conductors are respectively connected to the plurality of single ended data links for communicating the ultrasound echo data stream, and wherein unused host conductors of the plurality of host conductors are connected to quiet ground through a first termination resistor; and
**in that** the ultrasound probe comprises:
a plurality of probe conductors, wherein used probe conductors of the plurality of probe conductors are respectively connected to the plurality of single ended data links for communicating the ultrasound echo data stream, and wherein unused probe conductors of the plurality of probe conductors are connected to a voltage source through a second termination resistor.

2. The ultrasound imaging system of claim 1, wherein the plurality of host conductors comprise printed circuit board (PCB) traces, strip lines and/or micro-strip traces.

3. The ultrasound imaging system of claim 1, wherein the plurality of probe conductors comprise printed circuit board (PCB) traces, strip lines and/or micro-strip traces.

4. The ultrasound imaging system of claim 1, wherein used probe conductors of the plurality of probe conductors are connected to the voltage source through a third termination resistor.

5. The ultrasound imaging system of claim 4, wherein:
the first termination resistor comprises a 50 ohm resistor;
the second termination resistor comprises a 25 ohm resistor; and
the third termination resistor comprises a 50 ohm resistor.

6. The ultrasound imaging system of claim 5, wherein:
the plurality of single ended data links comprise 50 ohm coaxial cables;
the plurality of host conductors comprise 100 ohm conductors; and
the plurality of probe conductors comprise 50 ohm conductors.

7. The ultrasound imaging system of claim 1, wherein the cable assembly further comprises:
a plurality of AC couplers respectively connected to the plurality of host conductors in the host system, wherein the plurality of AC couplers are configured to maintain quiescent bias.

8. The ultrasound imaging system of claim 1, wherein the ultrasound probe comprises an application-specific integrated circuit (ASIC) connected to the cable assembly, and the host system comprises a field-programmable gate array (FPGA) connected to the cable assembly.

9. The ultrasound imaging system of claim 1, wherein the ultrasound probe comprises:
a transducer array comprising a plurality of transducer elements configured to emit the ultrasound signals into the medium, to receive ultrasound echo signals responsive to the ultrasound signals being reflected in the medium, and to output analog echo signals corresponding to the ultrasound echo signals;
a plurality of analog to digital converters (ADCs) coupled to the transducer array and configured to digitize the analog echo signals;
a multiplexer (MUX) coupled to the plurality of ADCs and configured to multiplex the digitized echo signals into the ultrasound echo data stream; and
a communication interface coupled to the MUX and configured to interface the MUX with the cable assembly for communicating the ultrasound echo data stream to the host system.

10. The ultrasound imaging system (100) of claim 1, further comprising:
the host system (130), wherein the host system is configured to receive the digital echo data stream from the ultrasound probe, and to generate and display ultrasound images from the digital echo data stream; and
wherein the cable assembly comprises a probe cable including the plurality of single ended coaxial cables for transmitting the ultrasound echo data stream from the ultrasound probe to the host system.

## Patentansprüche

1. Ultraschallbildgebungssystem (100), umfassend:
eine Ultraschallsonde (110), die konfiguriert ist, um einen digitalen Echodatenstrom zu erzeugen, der auf in einem Medium reflektierte Ultraschallsignale reagiert; **dadurch gekennzeichnet, dass** das System umfasst:
eine Kabelanordnung (150), die eine Vielzahl von einseitigen Datenverbindungen (155) umfasst, wobei die Kabelanordnung konfiguriert ist, um den Ultraschall-Echodatenstrom über die Vielzahl von einseitigen Datenverbindungen an ein Hostsystem (130) zu kommunizieren;
und dadurch, dass das Hostsystem umfasst:
eine Vielzahl von Host-Leitern, wobei verwendete Host-Leiter der Vielzahl von Host-Leitern jeweils mit der Vielzahl von einseitigen Datenverbindungen zum Kommunizieren des Ultraschall-Echodatenstroms verbunden sind, und wobei nicht verwendete Host-Leiter der Vielzahl von Host-Leitern über einen ersten Abschlusswiderstand mit Masse verbunden sind; und
dadurch, dass die Ultraschallsonde umfasst:
eine Vielzahl von Sondenleitern, wobei verwendete Sondenleiter der Vielzahl von Sondenleitern jeweils mit der Vielzahl von einseitigen Datenverbindungen zum Kommunizieren des Ultraschall-Echodatenstroms verbunden sind, und wobei nicht verwendete Sondenleiter der Vielzahl von Sondenleitern über einen zweiten Abschlusswiderstand mit einer Spannungsquelle verbunden sind.

2. Ultraschallbildgebungssystem nach Anspruch 1, wobei die Vielzahl von Host-Leitern Leiterplatten-, (PCB)-Leiterbahnen, -Streifenleitungen und/oder - Mikrostreifenleiterbahnen umfassen.

3. Ultraschallbildgebungssystem nach Anspruch 1, wobei die Vielzahl von Sondenleitern Leiterplatten-, (PCB)-Leiterbahnen, -Streifenleitungen und/oder - Mikrostreifenleiterbahnen umfassen.

4. Ultraschallbildgebungssystem nach Anspruch 1, wobei die verwendeten Sondenleiter der Vielzahl von Sondenleitern über einen dritten Abschlusswiderstand mit der Spannungsquelle verbunden sind.

5. Ultraschallbildgebungssystem nach Anspruch 4, wobei:
der erste Abschlusswiderstand einen 50-Ohm-Widerstand umfasst;
der zweite Abschlusswiderstand einen 25-Ohm-Widerstand umfasst; und
der dritte Abschlusswiderstand einen 50-Ohm-Widerstand umfasst.

6. Ultraschallbildgebungssystem nach Anspruch 5, wobei:
die Vielzahl von einseitigen Datenverbindungen 50-Ohm-Koaxialkabel umfassen;
die Vielzahl von Host-Leitern 100-Ohm-Leiter umfassen; und
die Vielzahl von Sondenleitern 50-Ohm-Leiter umfassen.

7. Ultraschallbildgebungssystem nach Anspruch 1, wobei die Kabelanordnung weiter umfasst:
eine Vielzahl von Wechselstromkopplern, die jeweils mit der Vielzahl von Host-Leitern im Host-System verbunden sind, wobei die Vielzahl von Wechselstromkopplern konfiguriert ist, um eine Ruhevorspannung aufrecht zu erhalten.

8. Ultraschallbildgebungssystem nach Anspruch 1, wobei die Ultraschallsonde einen anwendungsspezifischen integrierten Schaltkreis (ASIC) umfasst, der mit der Kabelanordnung verbunden ist, und das Hostsystem ein feldprogrammierbares Gate-Array (FPGA) umfasst, das mit der Kabelanordnung verbunden ist.

9. Ultraschallbildgebungssystem nach Anspruch 1, wobei die Ultraschallsonde umfasst:
ein Wandler-Array, das eine Vielzahl von Wandlerelementen umfasst, die konfiguriert sind, um Ultraschallsignale in das Medium auszusenden, Ultraschall-Echosignale als Reaktion auf die im Medium reflektierten Ultraschallsignale zu empfangen, und analoge Echosignale, die den Ultraschall-Echosignalen entsprechen, auszugeben;
eine Vielzahl von Analog-Digital-Wandlern (ADCs), die mit dem Wandler-Array gekoppelt sind und konfiguriert sind, um die analogen Echosignale digitalisieren;
einen Multiplexer (MUX), der mit der Vielzahl von ADCs gekoppelt ist und konfiguriert ist, um die digitalisierten Echosignale in den Ultraschall-Echodatenstrom zu multiplexen; und
eine Kommunikationsschnittstelle, die mit dem MUX gekoppelt ist und konfiguriert ist, um den MUX mit der Kabelanordnung zu verbinden, um den Ultraschall-Echodatenstrom dem Hostsystem zu kommunizieren.

10. Ultraschallbildgebungssystem (100) nach Anspruch 1, weiter umfassend:
das Hostsystem (130), wobei das Hostsystem konfiguriert ist, um den digitalen Echodatenstrom von der Ultraschallsonde zu empfangen und aus dem digitalen Echodatenstrom Ultraschallbilder zu generieren und anzuzeigen; und
wobei die Kabelanordnung ein Sondenkabel umfasst, das die Vielzahl von einseitigen Koaxialkabeln zum Übertragen des Ultraschall-Echodatenstroms von der Ultraschallsonde zum Hostsystem beinhaltet.

## Revendications

1. Système d'imagerie ultrasonore (100), comprenant :
une sonde ultrasonore (110) configurée pour générer un flux de données d'écho numérique en réponse aux signaux ultrasonores réfléchis dans un milieu ; **caractérisé en ce que** le système comprend :
un ensemble de câbles (150) comprenant une pluralité de liaisons de données à extrémité unique (155), dans lequel l'ensemble de câbles est configuré pour communiquer le flux de données d'écho ultrasonore à un système hôte (130) via la pluralité de liaisons de données à extrémité unique ;
et **en ce que** le système hôte comprend :
une pluralité de conducteurs hôtes, dans lequel les conducteurs hôtes utilisés parmi la pluralité de conducteurs hôtes sont connectés, respectivement, à la pluralité de liaisons de données à extrémité unique pour la communication du flux de données d'écho ultrasonore, et dans lequel les conducteurs hôtes inutilisés parmi la pluralité de conducteurs hôtes sont connectés à une masse silencieuse via une première résistance de terminaison ;
**en ce que** la sonde ultrasonore comprend :
une pluralité de conducteurs de sonde, dans laquelle les conducteurs de sonde utilisés parmi la pluralité de conducteurs de sonde sont connectés, respectivement, à la pluralité de liaisons de données à extrémité unique pour communiquer le flux de données d'écho ultrasonore, et dans laquelle les conducteurs de sonde inutilisés parmi la pluralité de conducteurs de sonde sont connectés à une source de tension via une deuxième résistance de terminaison.

2. Système d'imagerie ultrasonore selon la revendication 1, dans lequel la pluralité de conducteurs hôtes comprend des pistes de carte de circuit imprimé (PCB), des circuits à bande et/ou des pistes à micro-bandes.

3. Système d'imagerie ultrasonore selon la revendication 1, dans lequel la pluralité de conducteurs de sonde comprend des pistes de carte de circuit imprimé (PCB), des circuits à bande et/ou des pistes à micro-bandes.

4. Système d'imagerie ultrasonore selon la revendication 1, dans lequel les conducteurs de sonde utilisés parmi la pluralité de conducteurs de sonde sont connectés à la source de tension via une troisième résistance de terminaison.

5. Système d'imagerie ultrasonore selon la revendication 4, dans lequel :
la première résistance de terminaison comprend une résistance de 50 ohms ;
la deuxième résistance de terminaison comprend une résistance de 25 ohms ; et
la troisième résistance de terminaison comprend une résistance de 50 ohms.

6. Système d'imagerie ultrasonore selon la revendication 5, dans lequel :
la pluralité des liaisons de données à extrémité unique comprend des câbles coaxiaux de 50 ohms ;
la pluralité des conducteurs hôtes comprend des conducteurs de 100 ohms ; et
la pluralité des conducteurs de sonde comprend des conducteurs de 50 ohms.

7. Système d'imagerie ultrasonore selon la revendication 1, dans lequel l'ensemble de câbles comprend en outre :
une pluralité de coupleurs AC connectés, respectivement, à la pluralité de conducteurs hôtes dans le système hôte, dans lequel la pluralité de coupleurs AC sont configurés pour maintenir une polarisation de repos.

8. Système d'imagerie ultrasonore selon la revendication 1, dans lequel la sonde ultrasonore comprend un circuit intégré spécifique à l'application (ASIC) connecté à l'ensemble de câbles, et le système hôte comprend un réseau prédiffusé programmable par l'utilisateur (FPGA) connecté à l'ensemble de câbles.

9. Système d'imagerie ultrasonore selon la revendication 1, dans lequel la sonde ultrasonore comprend :
un réseau de transducteurs comprenant une pluralité d'éléments transducteurs configurés pour émettre les signaux ultrasonores dans le milieu, pour recevoir les signaux d'écho ultrasonores en réponse aux signaux ultrasonores réfléchis dans le milieu, et pour produire des signaux d'écho analogiques correspondant aux signaux d'écho ultrasonores ;
une pluralité de convertisseurs analogiques-numériques (ADC) couplés au réseau de transducteurs et configurés pour numériser les signaux d'écho analogiques ;
un multiplexeur (MUX) couplé à la pluralité de ADC et configuré pour multiplexer les signaux d'écho numérisés dans le flux de données d'écho ultrasonore ; et
une interface de communication couplée au MUX et configurée pour interfacer le MUX avec l'ensemble de câbles afin de communiquer le flux de données d'écho ultrasonore au système hôte.

10. Système d'imagerie ultrasonore (100) selon la revendication 1, comprenant en outre :
le système hôte (130), configuré pour recevoir le flux de données d'écho numérique provenant de la sonde ultrasonore, et pour générer et afficher des images ultrasonores à partir du flux de données d'écho numérique ;
dans lequel l'ensemble de câbles comprend un câble de sonde incluant la pluralité de câbles coaxiaux à extrémité unique pour transmettre le flux de données d'écho ultrasonore de la sonde ultrasonore au système hôte.
